# EUROPÄISCHE PATENTSCHRIFT

(11) **EP 1 733 693 B1**
(45) Veröffentlichungstag und Bekanntmachung des Hinweises auf die Patenterteilung: **23.04.2008**
(21) Anmeldenummer: 05012982.4
(22) Anmeldetag: 16.06.2005
(51) Int. Cl.: A61B 19/00

(54) **Medizintechnisches Trackingsystem mit Infrarotdatenübertragung**
Tracking system for medical equipment with infrared transmission
Système de localisation et de suivi pour équipement médical avec transmission infrarouge

(43) Veröffentlichungstag der Anmeldung: 20.12.2006
(73) Patentinhaber: BrainLAB AG, 85551 Kirchheim/Heimstetten (DE)
(72) Erfinder: Goldbach, Günter, 85661 Forstinning (DE)
(74) Vertreter: Schwabe - Sandmair - Marx

(56) Entgegenhaltungen:
- DE-A1- 19 639 615
- US-A- 6 161 033
- US-A1- 2002 147 455
- US-B1- 6 675 040

## Beschreibung

Die Erfindung betrifft das Gebiet der medizintechnischen Trackingsysteme sowie die Datenübertragung zwischen medizintechnischen Geräten.

Medizintechnische Trackingsysteme werden dazu verwendet, Instrumente oder Behandlungseinrichtungen in einem Raum in ihrer dreidimensionalen Position zu verfolgen bzw. zu bestimmen. Meist weisen sie zwei Kameras auf, und spezielle Trackingsysteme arbeiten mit unsichtbarem Licht, also beispielsweise mit Infrarotlichtkameras, welche Infrarotlicht erfassen. Das Infrarotlicht wird dabei durch eine systemeigene Infrarotlichtquelle bereitgestellt, meist in Form von kurzen Infrarotlichtblitzen. Diese Blitze werden von Referenz-Reflektoreinrichtungen an Instrumenten, Patienten oder Behandlungseinrichtungen reflektiert, und daraus wird die dreidimensionale Position der Instrumente bzw. Einrichtungen mittels der Kameraanordnung (und integrierter Elektronik) bestimmt. Aus der DE 196 39 615 A1 und der US 6,351,659 B1 sind Beispiele für ein solches Infrarot-Trackingsystem bekannt.

In Operationssälen werden viele ansteuerbare Geräte verwendet; um nur wenige Beispiele zu nennen: Höhenverstellbare Patientenliegen, Audio- und Videoaufzeichnungsgeräte oder OP-Lampen etc.. Außerdem findet ein Datentausch zwischen Geräten in einem Operationssaal statt, und ein Beispiel hierfür ist die Kabel-Übertragung von Positionsdaten zwischen den Trackingsystemen und einem medizintechnischen Navigationssystem.

Was die Ansteuerung von Geräten in einem Operationssaal betrifft, wird diese oftmals durch Infrarot-Fernbedienungen realisiert. Gerade in Operationssälen, wo ein Infrarot-Trackingsystem vorhanden ist, das mit sehr starken Infrarotsignalen arbeitet, können die Infrarot-Trackingsignale aber die Ansteuerungssignale aus den Fernbedienungen überlagern und auf diese Weise auch die Ansteuerungsfunktion beeinträchtigen. Außerdem kann eine Sättigung der Infrarot-Empfänger der anzusteuernden Geräte eintreten.

Es ist eine Aufgabe der vorliegenden Erfindung, die Geräteansteuerung bzw. die Datenübertragung im Umfeld eines medizintechnischen Trackingsystems zu optimieren.

Diese Aufgabe wird durch ein Kombinationssystem gemäß dem Anspruch 1 sowie durch ein Verfahren gemäß dem Anspruch 5 gelöst. Die Unteransprüche definieren vorteilhafte Ausführungsformen der vorliegenden Erfindung.

Die Erfindung stellt ein Kombinationssystem zur Verfügung, mit einem medizintechnischen Trackingsystem, das eine Kameraanordnung und eine Infrarotlichtquelle aufweist, und mit mindestens einem Gerät, das im medizintechnischen Umfeld (z.B. in einem Behandlungs- und/oder Untersuchungsraum bzw. -Umfeld) verwendet wird, insbesondere einem medizintechnischen Gerät, und das über Infrarotsignale angesteuert wird oder mittels Infrarotsignalen Daten austauscht, wobei die Infrarotlichtquelle des Trackingsystems mindestens einen Teil der kabellosen Ansteuerungs- bzw. Datenaustauscheinheit des Systems bildet. Mit anderen Worten wird die Infrarotlichtquelle in die Ansteuerung der Geräte bzw. in den Datentausch einbezogen bzw. zu einem Ansteuerungs- oder Datenaustauschelement gemacht. Dabei erreicht man vielerlei Vorteile, nämlich unter anderem, dass die Infrarotlichtquelle nicht mehr als Störfaktor, sondern als ein Teil des Ansteuerungs-/Datenübertragungssystems arbeitet und damit Störungen ausgeschlossen werden können. Andererseits werden beispielsweise auch Steuersignal- bzw. Datenübertragungen sicherer und zuverlässiger, weil die Infrarotlichtquelle des Trackingsystems relativ stark ist. Weitere Vorteile werden noch im Folgenden beschrieben.

Bei Ausführungsvarianten des erfindungsgemäßen Systems ist dieses dadurch gekennzeichnet, dass das oder die medizintechnische(n) Gerät(e) mindestens eines der folgenden Geräte ist bzw. umfassen:
- eine Operationslampe;
- ein medizintechnisches Navigationssystem;
- ein medizintechnischer Roboter;
- ein Daten- oder Bildaufzeichnungsgerät;
- ein Daten- oder Bildwiedergabegerät;
- eine Patientenliege;
- ein Operationssaal-Mikroskop;
- ein bipolares oder unipolares, chirurgisches Hochfrequenz-Instrument;
- ein Endoskop;
- eine Ultraschall-Vorrichtung;
- ein Fluoroskop;
- eine Laser-Registrierungsvorrichtung;
- mehrfache Trackingeinrichtungen;
- automatische Träger- oder Halterungsvorrichtungen;
- ein Deckenlicht;
- ein Audio/Videosystem;
- eine Wärmematte;
- einen Drucker; oder
- jedwedes andere ansteuerbare oder datenverarbeitungsfähige Gerät in einem Operationssaal.

Bei Operationslampen kann die Ansteuerung die Helligkeit, den Fokus oder die Position der Lampen betreffen. Die Mikroskopansteuerung kann den Fokus, den Zoom, die Position oder auch die Helligkeit der verwendeten Mikroskoplampe beeinflussen. Chirurgische Hochfrequenz-Instrumente können beispielsweise Schneid- oder Koagulationsinstrumente sein, die angesteuert werden. Bei Endoskopen können Lichtquellen ausgewählt und in ihrer Helligkeit eingestellt werden. Ultraschallgeräte können Einstellungen bezüglich des Fokus', der Tiefe, der Frequenz oder der Modi benötigen. C-Bogen-Fluoroskope sind hinsichtlich der Dosis, der Zeit und der Auslösung einstellbar. Für Laser-Registrierungsvorrichtungen kann die verwendete Energie und das Timing eingestellt werden, und multiple Trackingsysteme können bezüglich ihrer Synchronisation und der Übernahmezeitpunkte eingestellt werden. Automatische Trägersysteme können Ansteuerungen hinsichtlich der Geschwindigkeit und Richtung erfahren, ebenso wie Roboter, die noch Positions- und Modusinformationen erhalten können.

Aufnahme- und Abspielgeräte können die üblichen Steuerbefehle erhalten, aber auch Daten, die sie aufzeichnen sollen. Eingabe- und Steuerungsgeräte bzw. Bildausgabegeräte oder solche Geräte in kombinierter Form (z.B. berührungsempfindlicher Bildschirm) können alle möglichen Eingabe- und Ausgabefunktionen sowie Datenaustauschfunktionen erfüllen und dabei die Steuerungs- bzw. Datenaustauschfunktion gemäß der vorliegenden Erfindung nutzen. Die Temperatur von Wärmematten kann eingestellt werden, und es können Ausgabesysteme angesteuert bzw. mit Daten versorgt werden wie zum Beispiel Drucker, um Dokumentationszwecken zu dienen. All diese Funktionen können über die ohnehin vorhandene Infrarotlichtquelle erfüllt werden.

Bevorzugt weist das System eine zentrale Verarbeitungseinheit für die Ansteuerung bzw. den Datenaustausch auf, die vorzugsweise in einem der medizinischen Geräte integriert ist. Beispielsweise kann die Datenverarbeitung durch ein vorhandenes Navigationssystem oder einen Teil davon erfolgen.

Ebenfalls ist es im Rahmen der Erfindung möglich, eine zentrale Eingabeeinheit für die Ansteuerung bzw. den Datenaustausch bereitzustellen, sozusagen als Steuerkonsole. Diese zentrale Eingabeeinheit ist ebenfalls vorzugsweise in einem der medizinischen Geräte integriert, insbesondere in dem Gerät mit der oben genannten Verarbeitungseinheit. Es kann sich beispielsweise um einen berührungsempfindlichen Bildschirm des Navigationssystems handeln.

Bei dem erfindungsgemäßen Verfahren zur Ansteuerung medizintechnischer Geräte bzw. zum Datentausch zwischen medizintechnischen Geräten wird eine Infrarotlichtquelle eines medizintechnischen Trackingsystems als mindestens ein Teil der kabellosen Ansteuerungs- bzw. Datenaustauscheinheit des Systems verwendet.

Ausführungsformen des erfindungsgemäßen Verfahrens umfassen das Ansprechen der oben aufgelisteten Geräte durch die Infrarotlichtquelle, wobei der Begriff "Ansprechen" jedweden Datentausch, also Informationsaustausch (z.B. Bilder, medizinische oder anatomische Daten) und Steuersignalaustausch umfasst.

Die Ansteuerungs- bzw. Datentauschsignale können in den Signalpausen zwischen den Trackingsignalabgaben der Infrarotlichtquelle abgegeben werden. Ferner oder zusätzlich ist es möglich, die Datentauschsignale als Ergänzung zu den Trackingsignalabgaben der Infrarotlichtquelle oder anstelle der Trackingsignalabgaben zu versenden bzw. abzugeben.

Gemäß einer bevorzugten Ausführungsform des erfindungsgemäßen Verfahrens wird die Kameraanordnung so angesteuert, dass sie zu den Zeiten, wo keine Trackingsignalabgaben erfolgen, nicht aufnahmebereit ist. Dadurch wird vermieden, dass Ansteuerungs- oder Datentausch-Signale unerwünschterweise für das Instrumententracking verwendet werden.

Die Erfindung betrifft ferner ein Programm, das, wenn es auf einem Computer läuft oder in einem Computer geladen ist, den Computer veranlasst, eines der oben beschriebenen Verfahren durchzuführen. Sie umfasst auch ein Computerprogramm-Speichermedium, das ein solches Programm aufweist.

Mit der vorliegenden Erfindung wird ein Steuerungs- bzw. Datenübertragungssystem bereitgestellt, bei dem die schon integrierte Infrarot-Signalabgabefähigkeit des Trackingsystems verwendet wird, um andere infrarotsteuerbare Vorrichtungen durch die Emulation ihrer Befehle anzusteuern. Hierzu ist es durchaus akzeptabel, den normalen Instrumententrackingmodus anzuhalten und/oder geeignete Steuerbefehle während interner Verarbeitungszeiten zu senden, wo die IR-Lichtsignale nicht zur Bildaquirierung benötigt werden. Es ist zu bemerken, dass, wenn hierin von Infrarotsignalen gesprochen wird, andere kabellose Signalübertragungsarten, beispielsweise andere Arten, die mit nicht sichtbarem Licht arbeiten, ebenfalls von Fachleuten ins Auge gefasst werden könnten. Ganz allgemein wäre es beispielsweise auch möglich, Ultraviolett-Strahlung als Trackingsignal und gleichzeitig als Datenaustausch- bzw. Ansteuerungssignal zu verwenden. Es ist im Rahmen der Erfindung immer dann von Vorteil, wenn die vom Trackingsystem verwendete Energie auch die Energie zum Ansteuern von Geräten bzw. zum Austauschen von Daten ist.

Die gesamten Daten, die von dem Trackingsystem selbst zur Verfügung gestellt werden, können entweder über digitale oder über analoge Infrarotlicht-Modulation an eine oder mehrere Host-Vorrichtungen kabellos gesendet werden (z.B. Computer, Roboter, Navigationssystem), ohne dass zusätzliche kabellose Übertragungssysteme und ―vorrichtungen benötigt werden.

Das praktische Problem, mehrere Infrarot-Steuerungseinheiten im Operationssaal bereitstellen zu müssen, um unterschiedliche Vorrichtungen anzusteuern, wird durch ein erfindungsgemäßes Zentralsteuerungssystem überwunden. Es werden nicht mehr mehrere Fernbedienungen benötigt und notwendige Einstellungen an den Geräten können von der zentralen Steuerungseinheit (z.B. berührungsempfindlicher Bildschirm) aus vorgenommen werden.

Probleme mit der unterbrochenen "line of sight (Sichtlinie)", also mit verstellten Ansteuerungswegen, die bei Hand-Fernbedienungen auftreten, werden eliminiert, weil die Infrarotblitze des Trackingsystems so stark sind, dass sie praktisch jeden Winkel in dem Operationssaal erreichen können und zwar entweder direkt oder über ausreichend starke Lichtreflexionen im Raum. Batterien für die Infrarot-Fernbedienungen werden nicht mehr benötigt; durch den Wegfall der Fernbedienungen und ihrer Batterien wird auch die Erhaltung der Sterilität sehr viel einfacher.

Mit der Erfindung wird die kabellose Datenübertragung an mehrere Empfänger möglich, und zwar immer ohne im Operationssaal störende Kabel und mit sehr geringen Integrationskosten, weil mindestens der Sender, also die Infrarotlichtquelle, schon vorhanden ist. Es ist eine vollständige Integration von mehreren Geräten im Operationssaal ohne galvanische Schnittstellen möglich.

Die Erfindung wird im Weiteren anhand von Ausführungsformen näher erläutert. Im Rahmen der Erfindung können die hierin beschriebenen Merkmale einzeln oder in jedweder Kombination realisiert werden. In den Zeichnungen zeigen:
- Figur 1: ein Operationssaal-Setup mit dem erfindungsgemäßen System;
- und Figur 2: eine typische Infrarot-Signalabgabesequenz für ein passives Trackingsystem bzw. Navigationssystem (Image-Guided-Surgery System).

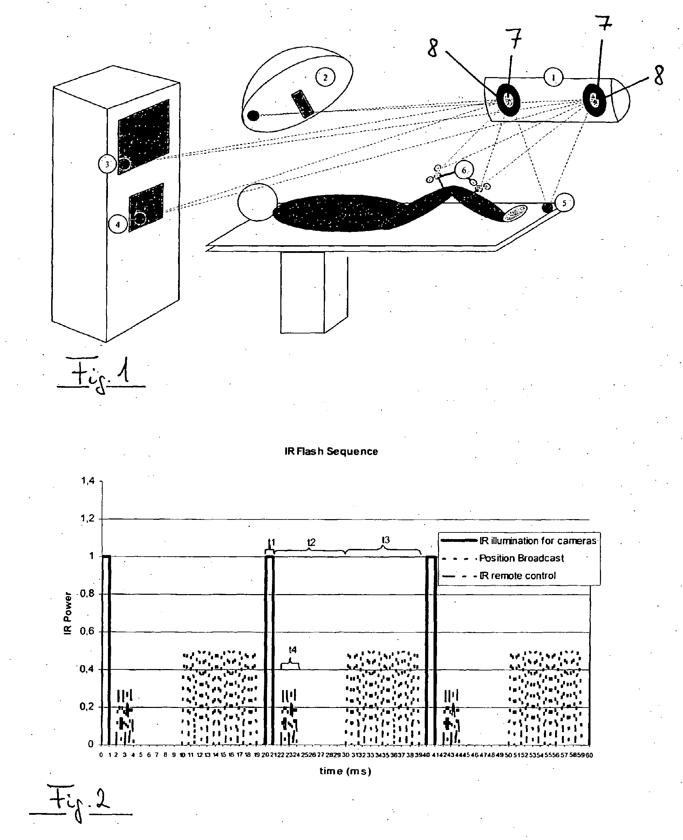

In der Figur 1 ist mit dem Bezugszeichen 1 ein medizintechnisches Trackingsystem bezeichnet. Es weist zwei Infrarotkameras 7 auf, die von Diodenringen 8 umgeben sind. Die Diodenringe 8 enthalten Infrarot-Dioden, und sie können Infrarot-Blitze bzw. ―Signale aussenden, deren Reflexionen dann von den Kameras 7 (Infrarotkameras) empfangen werden können. Auf diese Weise wird herkömmlich zum Beispiel die Position eines Patienten-Körperteils "getrackt", also bestimmt und verfolgt. Dieses herkömmliche Tracking geschieht beispielsweise mit den Referenzsternen, die am dargestellten Patientenbein angebracht sind und das Bezugszeichen 6 tragen. Mit solchen Referenzsternen können auch medizinische Instrumente oder Behandlungseinrichtungen getrackt werden.

In dem Operationssaal-Setup der Figur 1 sind noch weitere Geräte dargestellt, nämlich eine Patientenliege 5, eine OP-Lampe 2, ein Navigationssystem 3 und eine Aufzeichnungsvorrichtung (Video/Audio) 4. Gemäß der Erfindung werden nun diese Geräte ebenfalls durch Signale angesteuert, die von den LED-Ringen 8 als Infrarotsignale abgegeben werden. Dazu sind an den Geräten 2, 3, 4 und 5 jeweils als Punkte dargestellte Empfänger (es können die schon vorhandenen IR-Empfänger der Geräte sein) vorhanden. Durch eine Signalabgabe über den IR-LED-Ring 8 an den Empfänger der Liege 5 kann beispielsweise durch deren Motoren ihre Höhe verstellt werden. Die Operationslampe 2 kann in einen anderen Modus geschaltet oder heller bzw. dunkler gemacht werden. Auch ihr Fokus kann verändert werden. Das Video/Audio-Aufzeichnungsgerät 4 kann angesteuert werden, um Aufzeichnungen zu beginnen oder auch Aufzeichnungen abzuspielen, die behandlungsunterstützend sein könnten.

Mit dem Navigationssystem 3 kann das Trackingsystem über die LED-Ringe 8 sowohl Ansteuerungsbefehle austauschen als auch Daten übertragen. Ein Beispiel für die Datenübertragung ist die Übermittlung der erfassten Positionen von Referenzsternen 6 durch das Trackingsystem 1 an das Navigationssystem 3. Es können aber auch Steuerungsbefehle übergeben werden, und anhand des Navigationssystems lässt sich beispielsweise darstellen, dass dies nicht nur in Richtung vom Trackingsystem 1 weg erfolgen muss. Das Navigationssystem kann beispielsweise einen hier nicht dargestellten berührungsempfindlichen Bildschirm aufweisen, über den Steuerungseingaben möglich sind. Das Navigationssystem sendet diese Steuerungseingaben dann an das Trackingsystem 1 (an die Kameras oder einen zusätzlichen Empfänger), welches wiederum die Steuerbefehle über die LED-Ringe 8 weitergibt. Es können aber auch Steuerbefehle vom Navigationssystem 3 an das Trackingsystem 1 übertragen werden, die lediglich die Ansteuerung des Trackingsystems, d.h. die Änderung von variierbaren Eigenschaften des Trackingsystems 1, betreffen (z.B. Kameraausrichtung).

Damit entsteht durch viele schon vorhandene Elemente durch die vorliegende Erfindung ein optimales Kommunikationssystem für ein OP-Setup.

Die Figur 2 stellt die typische Infrarot-Signalabgabesequenz eines passiven Image-Guided-Surgery-Systems (Navigationssystems) dar. Die Hochachse zeigt die Infrarot-Energie, nach rechts ist die Zeit in Millisekunden aufgetragen. Die dicken Linien bezeichnen die Infrarot-Signalabgabe für die Trackingkamera, die kurzgestrichelten Einzeichnungen betreffen die Positions-Informationsweitergabe und die strichpunktierten Linien zeigen die Infrarot-Fernbedienungs- bzw. Steuerungs-/Datentauschzeiten an.

In der Darstellung bezeichnet t1 die Bildaquirierungszeit. Die Hauptaufgabe der IR-Blitze für ein passives Trackingsystem basierend auf IR-Technologie ist es, die Szene des Operationssaals mit starkem Infrarotlicht zu beleuchten, während die Sensoren (Kameras 7) die Bilder für die Marker-Positionserfassung aquirieren (z.B. Marker-Referenzänordnungen 6 in Figur 1). Dies dauert typischerweise von einigen 10 µs bis zu einigen Millisekunden. In der Figur 2 wird alle 20 Millisekunden eine Millisekunde benötigt, was für ein Trackingsystem mit 50 Bildern pro Sekunde der Fall wäre.

Die Zeit t2 ist die Bildverarbeitungszeit: Anschließend an die Bildaquirierungszeit t1 benötigt das Bildverarbeitungssystem eine gewisse Zeit, um die 3D-Positionen zu erfassen und zu verarbeiten.

Die Zeit t3 ist die Datenübergabezeit bzw. die Datenweitergabezeit: Wenn die Bildverarbeitung erledigt ist, können die Positionsinformationen unter Verwendung der gleichen IR-Strahler (LED-Ringe 8 in Figur 1) übertragen werden (zum Navigationssystem), und zwar mit derselben oder sogar einer niedrigeren Energie wie die IR-Blitze (t1) unter Verwendung optischer Datenübertragungstechniken, zum Beispiel IrDA.

Die Zeit t4 verbleibt als freie Zeit zur Ansteuerung anderer infrarotsteuerbarer Geräte. Während der Bildverarbeitungszeit t2 können die IR-Strahler (LED-Ringe 8 in Figur 1) verwendet werden, um andere Geräte in der Nähe anzusteuern, indem deren Infrarot-Fernbedienungsprotokolle emuliert werden.

Unter Verwendung dieser Technik wird die Interferenz anderer Infrarot-Fernbedienungen, die im Setup zusammen mit der IR-Ausleuchtung des Trackingsystems verwendet werden müssten, durch ein Zeit-Multiplexing (Zeit-Phäsen-Steuerung) vermieden. Die relativ lange Bildverarbeitungszeit kann als Zeitfenster zur Steuerung anderer infrarotsteuerbarer Geräte verwendet werden, da sie ansonsten ungenutzt verbleibt. Die Daten des Navigationssystems können an einen oder mehrere Empfänger weitergeleitet werden, welche die Trackinginformation auch weiter verarbeiten können.

## Patentansprüche

1. Kombinationssystem mit
a) einem medizintechnischen Trackingsystem (1), das eine Kameraanordnung (7) und eine Infrarotlichtquelle (8) aufweist, und mit
b) mindestens einem Gerät (2, 3, 4, 5), das im medizintechnischen Umfeld verwendet wird, insbesondere einem medizintechnischen Gerät,
**dadurch gekennzeichnet, dass**
c) das Gerät über Infrarotsignale angesteuert wird und die Infrarotlichtquelle (8) des Trackingsystems (1) mindestens einen Teil einer Kabellosen Ansteuerungseinheit des Systems bildet.

2. System nach Anspruch 1, **dadurch gekennzeichnet, dass** das Gerät mittels der Infrarotsignale Daten austauscht.

3. System nach Anspruch 1 oder 2, **dadurch gekennzeichnet, dass** das oder die medizintechnische(n) Gerät(e) mindestens eines der folgenden Geräte ist bzw. umfassen:
- eine Operationslampe (2);
- ein medizintechnisches Navigationssystem (3);
- ein medizintechnischer Roboter;
- ein Daten- oder Bildaufzeichnungsgerät (4);
- ein Daten- oder Bildwiedergabegerät;
- eine Patientenliege (5);
- ein Operationssaal-Mikroskop;
- ein bipolares oder unipolares, chirurgisches Hochfrequenz-Instrument;
- ein Endoskop;
- eine Ultraschall-Vorrichtung;
- ein Fluoroskop;
- eine Laser-Registrierungsvorrichtung;
- mehrfache Trackingeinrichtungen;
- automatische Träger- oder Halterungsvorrichtungen;
- ein Deckenlicht;
- ein AudioNideosystem;
- eine Wärmematte;
- einen Drucker; oder
- jedwedes andere ansteuerbare oder datenverarbeitungsfähige Gerät in einem Operationssaal.

4. System nach Anspruch 1, 2 oder 3, **dadurch gekennzeichnet, dass** es eine zentrale Verarbeitungseinheit für die Ansteuerung bzw. den Datenaustausch aufweist, die vorzugsweise in einem der medizintechnischen Geräte (2, 3, 4, 5) integriert ist.

5. System nach einem der Ansprüche 1 bis 4, **dadurch gekennzeichnet, dass** es eine zentrale Eingabeeinheit für die Ansteuerung bzw. den Datenaustausch aufweist, die vorzugsweise in einem der medizintechnischen Geräte (2, 3, 4, 5) integriert ist, insbesondere in dem Gerät mit der Verarbeitungseinheit.

6. Verfahren zur Ansteuerung von Geräten (2, 3, 4, 5) im medizintechnischen Umfeld **dadurch gekennzeichnet, dass** eine Infrarotlichtquelle (8) eines medizintechnischen Trackingsystems (1) als mindestens ein Teil einer kabellosen Ansteuerungseinheit für die Geräte verwendet wird.

7. Verfahren nach Anspruch 6, bei dem die Infrarotlichtquelle zum Datentausch zwischen solchen Geräten (2, 3, 4, 5) verwendet wird.

8. Verfahren nach Anspruch 6 oder 7, bei dem mittels der Infrarotlichtquelle mindestens eines der Geräte angesprochen wird, die in Anspruch 2 aufgelistet sind.

9. Verfahren nach Anspruch 6, 7 oder 8, bei dem die Ansteuerungs- bzw. Datentauschsignale in den Signalpausen zwischen den Trackingsignalabgaben der Infrarotlichtquelle abgegeben werden.

10. Verfahren nach einem der Ansprüche 6 bis 9, bei dem die Ansteuerungs- bzw. Datentauschsignale zusätzlich zu den Trackingsignalabgaben der Infrarotlichtquelle oder anstelle der Trackingsignalabgaben abgegeben werden.

11. Verfahren nach einem der Ansprüche 6 bis 10, bei dem die Kameraanordnung (7) so angesteuert wird, dass sie zu den Zeiten, wo keine Trackingsignalabgaben erfolgen, nicht aufnahmebereit ist.

12. Programm, das, wenn es auf einem Computer läuft oder in einem Computer geladen ist, den Computer veranlasst, ein Verfahren gemäß einem der Ansprüche 6 bis 11 durchzuführen.

13. Computerprogramm-Speichermedium, das ein Programm nach Anspruch 12 aufweist.

## Claims

1. A combined system, comprising:
a) a medical tracking system (1) comprising a camera array (7) and an infrared light source (8); and comprising
b) at least one appliance (2, 3, 4, 5) which is used in a medical setting, in particular a medical appliance,
**characterised in that**
c) the appliance is controlled via infrared signals, and the infrared light source (8) of the tracking system (1) forms at least a part of a wireless control unit of the system.

2. The system according to claim 1, **characterised in that** the appliance exchanges data by means of the infrared signals.

3. The system according to claim 1 or 2, **characterised in that** the medical appliance(s) are and/or include at least one of the following appliances:
- an operation lamp (2);
- a medical navigation system (3);
- a medical robot;
- a data or image recording appliance (4);
- a data or image reproducing appliance;
- a patient couch (5);
- an operating theatre microscope;
- a bi-polar or uni-polar, surgical high-frequency instrument;
- an endoscope;
- an ultrasound device;
- a fluoroscope;
- a laser registration device;
- multiple tracking means;
- automatic supporting or holding devices;
- a ceiling light;
- an audio/video system;
- a thermal mat;
- a printer; or
- any other appliance which can be controlled or can process data in an operating theatre.

4. The system according to claim 1, 2 or 3, **characterised in that** it comprises a central processing unit for controlling and/or data exchange which is preferably integrated in one of the medical appliances (2, 3, 4, 5).

5. The system according to any one of claims 1 to 4, **characterised in that** it comprises a central input unit for controlling and/or data exchange which is preferably integrated in one of the medical appliances (2, 3, 4, 5), in particular in the appliance comprising the processing unit.

6. A method for controlling appliances (2, 3, 4, 5) in a medical setting, **characterised in that** an infrared light source (8) of a medical tracking system (1) is used as at least a part of a wireless control unit for the appliances.

7. The method according to claim 6, wherein the infrared light source is used for data exchange between such appliances (2, 3, 4, 5).

8. The method according to claim 6 or 7, wherein by means of the infrared light source, at least one of the appliances listed in claim 3 is addressed.

9. The method according to claim 6, 7 or 8, wherein the control signals and/or data exchange signals are outputted in the signal intervals between the tracking signal outputs of the infrared light source.

10. The method according to any one of claims 6 to 9, wherein the control signals and/or data exchange signals are outputted in addition to the tracking signal outputs of the infrared light source or instead of the tracking signal outputs.

11. The method according to any one of claims 6 to 10, wherein the camera array (7) is controlled such that it is not ready to record at the times when there are no tracking signal outputs.

12. A program which, when running on a computer or loaded onto a computer, causes the computer to carry out a method in accordance with any one of claims 6 to 11.

13. A computer program storage medium comprising a program according to claim 12.

## Revendications

1. Système combiné avec
a) un système de poursuite médical (1) qui comporte un ensemble caméra (7) et une source de lumière infrarouge (8), et avec
b) au moins un appareil (2, 3, 4, 5) qui est utilisé dans le domaine médical, en particulier un appareil médical,
**caractérisé en ce que**
c) l'appareil est commandé par des signaux infrarouges et la source de lumière infrarouge du système de poursuite (1) forme au moins une partie d'une unité de commande sans fil du système.

2. Système selon la revendication 1, **caractérisé en ce que** l'appareil échange des données au moyen des signaux infrarouges.

3. Système selon la revendication 1 ou 2, **caractérisé en ce que** le ou les appareil(s) médical(médicaux) sont au moins l'un des appareils suivants ou comprennent
- une lampe pour opération (2) ;
- un système de navigation médical (2) ;
- un robot médical ;
- un appareil d'enregistrement de données ou d'images (4) ;
- un appareil de lecture de données ou d'images ;
- un lit pour patient (5) ;
- un microscope pour salle d'opération ;
- un instrument chirurgical à haute fréquence, bipolaire ou unipolaire ;
- un endoscope ;
- un dispositif à ultrasons ;
- un fluoroscope ;
- un dispositif d'enregistrement laser ;
- des dispositifs multiples de poursuite ;
- des dispositifs automatiques de support ou de fixation ;
- un plafonnier ;
- un système audio/vidéo ;
- un matelas chauffant ;
- une imprimante ; ou
- tout autre appareil commandable ou pouvant traiter des données dans une salle d'opération.

4. Système selon la revendication 1, 2 ou 3, **caractérisé en ce qu'**il comporte une unité de traitement centrale pour la commande ou l'échange des données, qui est de préférence intégrée dans l'un des appareils médicaux (2, 3, 4, 5).

5. Système selon l'une des revendications 1 à 4, **caractérisé en ce qu'**il comporte une unité de saisie centrale pour la commande d'échange des données, qui est intégrée de préférence dans l'un des appareils médicaux (2, 3, 4, 5), en particulier dans l'appareil avec l'unité de traitement.

6. Procédé de commande d'appareil (2, 3, 4, 5) dans le domaine médical, **caractérisé en ce qu'**on utilise aussi bien une source de lumière infrarouge (8) d'un système de poursuite médical (1) qu'au moins une partie d'une unité de commande sans fil pour les appareils.

7. Procédé selon la revendication 6, dans lequel on utilise la source de lumière infrarouge pour l'échange de données entre ces appareils (2, 3, 4, 5).

8. Procédé selon la revendication 6 ou 7, dans lequel on sollicite au moyen de la source de lumière infrarouge au moins l'un des appareils, tels qu'ils figurent dans la liste de la revendication 2.

9. Procédé selon l'une quelconque des revendications 6, 7 ou 8, dans lequel les signaux de commande ou d'échange des données sont délivrés dans les pauses des signaux entre les sorties de signaux de poursuite de la source de lumière infrarouge.

10. Procédé selon l'une quelconque des revendications 6 à 9, dans lequel les signaux de commande ou d'échange des données sont délivrés en supplément aux sorties de signaux de poursuite de la source de lumière infrarouge ou à la place des sorties de signaux de poursuite.

11. Procédé selon l'une quelconque des revendications 6 à 10, dans lequel l'ensemble caméra (7) est commandé de manière à ne pas être en veille aux moments auxquels aucun signal de poursuite n'est délivré.

12. Programme qui lorsqu'il tourne sur un ordinateur ou est chargé dans un ordinateur, amène l'ordinateur à exécuter un procédé selon l'une des revendications 6 à 11.

13. Support de mémoire de programme d'ordinateur qui comporte un programme selon la revendication 12.
